(19) Europäisches Patentamt / European Patent Office / Office européen des brevets

(11) **EP 3 180 610 B1**

(12) **EUROPÄISCHE PATENTSCHRIFT**

(45) Veröffentlichungstag und Bekanntmachung des Hinweises auf die Patenterteilung:
**13.04.2022 Patentblatt 2022/15**

(21) Anmeldenummer: **15756545.8**

(22) Anmeldetag: **10.08.2015**

(51) Internationale Patentklassifikation (IPC):
**G01N 33/38** $^{(2006.01)}$    **G01N 25/72** $^{(2006.01)}$
**G01N 3/60** $^{(2006.01)}$

(52) Gemeinsame Patentklassifikation (CPC):
**G01N 33/388;** G01N 3/60; G01N 25/72

(86) Internationale Anmeldenummer:
**PCT/AT2015/050194**

(87) Internationale Veröffentlichungsnummer:
**WO 2016/023055 (18.02.2016 Gazette 2016/07)**

(54) **VERFAHREN ZUR PRÜFUNG EINES KÖRPERS MIT SPRÖDEM MATERIALVERHALTEN**

METHOD FOR TESTING A BODY WITH BRITTLE MATERIAL BEHAVIOR

PROCÉDÉ DE TEST D'UN CORPS DONT LA MATIÈRE EST FRAGILE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.08.2014 AT 505602014**

(43) Veröffentlichungstag der Anmeldung:
**21.06.2017 Patentblatt 2017/25**

(73) Patentinhaber: **Materials Center Leoben Forschung GmbH**
**8700 Leoben (AT)**

(72) Erfinder:
• **STROBL, Stefan**
 **3432 RN Nieuwegein (NL)**
• **EBNER, Reinhold**
 **8793 Trofaiach (AT)**
• **SUPANCIC, Peter**
 **8700 Leoben (AT)**
• **DANZER, Robert**
 **8042 Graz (AT)**

(74) Vertreter: **Wirnsberger & Lerchbaum Patentanwälte OG**
**Mühlgasse 3**
**8700 Leoben (AT)**

(56) Entgegenhaltungen:
EP-A1- 0 872 725       DE-A1-102007 045 636
DE-A1-102010 017 351    DE-C1- 4 419 750
JP-A- H0 886 731

**Beschreibung**

Die Erfindung betrifft ein Verfahren zur Prüfung eines Körpers mit sprödem Materialverhalten, insbesondere eines keramischen Körpers, auf Einsatztauglichkeit, wobei der Körper zumindest bereichsweise einer Thermoschockbehandlung durch Abkühlung oder Erwärmung von einer ersten Temperatur auf eine zweite Temperatur unterworfen und vorhandene Risse detektiert werden, wonach auf Grundlage der detektierten Risse über die Einsatztauglichkeit des Körpers entschieden wird.

Keramische Körper finden aufgrund eines guten Eigenschaftsprofils vielfältigen Einsatz in verschiedenen Bereichen der Technik. Zu den besonders bevorzugten Eigenschaften keramischer Körper zählen insbesondere eine hohe Härte sowie ein exzellentes Verschleißverhalten. Dies macht keramische Körper beispielsweise interessant für einen Einsatz in Implantaten, Wälzlagern oder Schneidelementen sowie anderen Massenwaren. Oft sind diese keramischen Körper im Einsatz hohen Beanspruchungen im oberflächennahen Bereich ausgesetzt, gegebenenfalls auch bei hohen Temperaturen.

Keramische Körper können auch eingesetzt werden, wenn im Einsatz rasche Temperaturwechsel erfolgen, beispielsweise bei biegebeanspruchten Ventilen, Werkzeugen, Rohren oder anderen Teilen. Aber auch für Bauteile, bei welchen im Einsatz hohe lokale Druckbeanspruchungen gegeben sind, wie bei Wälz- oder Kugellagern, bewähren sich keramische Körper.

Inhärent weisen keramische Körper an den Oberflächen, aber auch im Inneren Risse auf, die insbesondere durch die Herstellung und/oder eine Nachbearbeitung, beispielsweise ein Schleifen, bedingt sein können. Vor allem bei einer Nachbearbeitung reagieren keramische Bauteile sehr empfindlich, weshalb keramische Bauteile grundsätzlich oftmals mit Rissen behaftet sind.

Durch einsatzbedingte Spannungen können Risse dazu führen, dass ein keramisches Bauteil versagt. Ein Riss kann unter Belastung wachsen und daher zu einem unerwünschten Materialversagen führen.

Im Rahmen der gegenständlichen Erfindung wurde erkannt, dass je nach Belastung zwischen unterkritischen und überkritischen Rissen unterschieden werden kann. Unterkritisch kleine Risse, die in der Regel bei allen keramischen Körpern bzw. Bauteilen vorhanden sind, breiten sich selbst bei einer Belastung im Einsatz nicht aus und sind daher nicht weiter kritisch. Hingegen können überkritisch lange Risse dazu führen, dass diese bei Belastung im Einsatz rasch stark wachsen und letztlich ein Versagen des Bauteils bewirken.

Es wäre wünschenswert, ein Prüfverfahren parat zu haben, das es auf einfache Weise ermöglicht, keramische Körper bzw. Bauteile auf eine Einsatztauglichkeit zu untersuchen.

Aus dem Stand der Technik, beispielsweise aus der JP H08 86731 A, sind für keramische Bauteile sogenannte Thermoschockverfahren bekannt geworden. Bei diesen Verfahren werden keramische Bauteile vor einem Einsatz zur Prüfung einer Thermoschockbehandlung unterzogen. Unter einer Thermoschockbehandlung wird im Rahmen der gegenständlichen Erfindung eine spontane, also in kurzer Zeit erfolgende Temperaturerhöhung oder Abkühlung eines keramischen Bauteils verstanden.

Eine Thermoschockbehandlung keramischer Bauteile ist beispielsweise aus der DE 10 2010 017 351 A1 bekannt, die ein spezielles Verfahren zur Prüfung von keramischen Bauteilen für Solarabsorber beschreibt, wobei die keramischen Bauteile innerhalb einer kurzen Zeit einer schnellen Erhitzung ausgesetzt werden, um fehlerhafte Bauteile gezielt zum Ausfall zu bringen. Eine Detektion von auftretenden Rissen erfolgt während eines nachfolgenden Abkühlens akustisch. Wird ein Wachsen eines Risses detektiert, wird das Bauteil ausgeschieden. Nachteilig dabei ist, dass der entsprechend ausgelegte Prüfversuch relativ unspezifisch ist. Abgesehen davon, dass sich dieser Versuch nicht für mechanisch hoch belastete Bauteile eignet, haftet diesem Prüfverfahren insbesondere der Nachteil an, dass auch Bauteile ausgeschieden werden, die eventuell brauchbar sein könnten, weil lediglich nicht kritische Risse detektiert werden und keine Differenzierung zwischen kritischen und nicht kritischen Rissen erfolgt.

Aufgabe der Erfindung ist es, ein Verfahren der eingangs genannten Art anzugeben, mit dem zuverlässig ein keramischer Körper auf Einsatztauglichkeit prüfbar ist und wobei das Verfahren so ausgelegt ist, dass auch eine Vielzahl von keramischen Körpern einer Prüfung unterzogen werden kann.

Diese Aufgabe mit dem im Anspruch 1 definierten Verfahren gelöst.

Ein mit der Erfindung erzielter Vorteil ist darin zu sehen, dass ein auch bei größeren Losgrößen 100%ig anwendbarer Überlastversuch (Proof-Test) angegeben wird, der sich zuverlässig zum Ausscheiden schadhafter Keramikteile, aber auch anderer Körper mit sprödem, insbesondere linear elastischem, Materialverhalten eignet, wobei ein Mindestwert einer Bauteilfestigkeit gewährleistet wird. Die Erfindung nutzt dabei unter anderem die Erkenntnis, dass eine Bruchfestigkeit eines Körpers mit linear elastischem Materialverhalten im Einsatz durch bereits vorhandene oberflächennahe oder im Inneren vorhandene Risse bestimmt wird. Ist eine zu erreichende Bruchfestigkeit des Körpers im Einsatz vorgegeben, ist dadurch über die Bruchzähigkeit des Materials auch eine kritische Rissgröße für den Einsatz bestimmt, die einen Schwellwert für die Thermoschockbehandlung darstellt.

Als Grundlage für die Auslegung einer Prüfung mittels Thermoschock für Materialien mit linear elastischem Materialverhalten wird vom Griffith/Irwin-Kriterium ausgegangen, wonach

$$K \geq K_{Ic} \ mit \ K = \sigma_{Ref} Y \sqrt{a\pi} \qquad (1),$$

wobei K für den Spannungsintensitätsfaktor steht, $K_{Ic}$ den kritischen Spannungsintensitätsfaktor darstellt, $\sigma_{Ref}$ für eine Referenzspannung in einer Probe ohne Riss steht, a für die Größe des Risses steht und Y einen geometrischen Faktor darstellt, mit welchem die Geometrie des Risses, des Spannungsfeldes und des Probenkörpers berücksichtigt wird. Unter Verwendung der Gleichung

$$\sigma_{th} = F \frac{\alpha E}{1-\nu} \Delta T \qquad (2)$$

für die thermisch induzierten Spannungen, in welcher $\sigma_{th}$ für die thermisch induzierten Spannungen und F für einen Faktor für die Bauteilgeometrie und die Quenchparameter steht, der zwischen 0 und 1 liegt, und wobei E für den Young-Modul, v für das Poisson-Verhältnis und $\Delta T$ für die Temperaturdifferenz bei der Thermoschockbehandlung steht, lässt sich K für den konkreten Versuch bei Vorliegen eines Risses mit der Länge a wie folgt berechnen:

$$K_{Ic} = F \frac{\alpha_{eff} E_{eff}}{1-\nu_{eff}} \Delta T_c Y \sqrt{a\pi} \qquad (3)$$

Zur Verlängerung eines Risses bzw. eines Defektes kommt es dann, wenn am Ort des Risses bzw. Defektes entsprechend Gleichung (3) die Spannungsintensität K die Bruchzähigkeit $K_{Ic}$ des Materials erreicht. Durch Umformulierung der vorstehenden Gleichung (3) ergibt sich für idealisierte Bedingungen wie temperaturunabhängige Materialparameter und geringe Spannungsänderungen über den Rissverlauf, dass eine bestimmte kritische Rissgröße bei vorgegebener Probengeometrie und geforderter (Mindest-)Bruchfestigkeit einer bestimmten kritischen Temperaturdifferenz $\Delta T_c$ bei der Thermoschockbehandlung entspricht. Erfindungsgemäß wird diese Temperaturdifferenz nummerisch berechnet.

Wird die Temperaturdifferenz entsprechend eingestellt, kann zuverlässig sichergestellt werden, dass alle der Thermoschockbehandlung mit der gegebenen Temperaturdifferenz standhaltenden bzw. positiv geprüften Körper im Einsatz dem Beanspruchungsprofil genügen. Durch die Prüfbedingungen wird dabei sichergestellt, dass bei dem Thermoschock Spannungsverteilungen auftreten, welche der Belastung im Einsatz möglichst nahe kommen. Körper, die im Prüfverfahren zu unzulässig langen Rissen geführt haben, die im Einsatz weiterwachsen könnten, können hingegen ausgeschieden werden. Zweckmäßigerweise ist vorgesehen, dass die Temperaturdifferenz so gewählt wird, dass die Bruchfestigkeit des Körpers im Einsatz mit einer Toleranz überschritten wird. Vorzugsweise ist diese Toleranz gering, sodass knapp oberhalb der finalen Belastung geprüft wird. Ist beispielsweise an der Oberfläche eine Bruchfestigkeit von 800 MPa gefordert, kann dieser Wert für die Zwecke der Prüfung beispielsweise auf 880 MPa erhöht werden, um zusätzlich einen Toleranzspielraum zur Verfügung zu haben. Damit ist sichergestellt, dass nur Körper eingesetzt werden, die bereits auf eine höhere Belastung geprüft worden sind und dieser standgehalten haben. Das Toleranzfenster nach oben beträgt üblicherweise maximal 20 %, bevorzugt maximal 15 %, insbesondere maximal 10 %, damit der Körper im Prüfverfahren nicht deutlich über die Belastung im Einsatz hinaus beansprucht wird. Dies stellt sicher, dass die Prüfbedingungen, mit einer zusätzlichen Sicherheit, den Einsatzbedingungen entsprechen.

Obwohl es nicht zwingend ist, hat es sich für bestimmte Anwendungen als günstig erwiesen, wenn der Körper so lange auf die erste Temperatur erwärmt wird, bis dieser in erwärmten Bereichen homogen die erste Temperatur aufweist, und anschließend rasch abgekühlt wird. Dadurch ergeben sich vergleichsweise einfache geometrische Rahmenbedingungen für die Kalkulation der minimalen Temperaturdifferenz und eine einfache Verfahrensführung durch Quenchen. Gleichwohl ist es auch möglich, dass in einer Verfahrensvariante auf die Einstellung einer konstanten bzw. homogenen ersten Temperatur im zum prüfenden Körper verzichtet wird. In diesem Fall wird lediglich ein oberflächennaher Bereich auf die erste Temperatur erwärmt und anschließend rasch abgekühlt. Analoges gilt, wenn die Thermoschockbehandlung aus einem raschen Aufheizen besteht. Ein rasches Aufheizen kommt insbesondere bei Bauteilen infrage, bei welchen einsatzbedingt innenliegende Zugspannungen auftreten. Dadurch treten im äußeren Bereich Druckspannungen und im Inneren Zugspannungen auf. Sofern nur eine im Einsatz speziell beanspruchte Stelle eines Bauteils zu prüfen ist, kann es unabhängig von der Temperaturführung auch ausreichend sein, nur diese der Thermoschockbehandlung zu unterwerfen. Beispielsweise kann ein bereichsweises rasches Aufheizen mit einem leistungsstarken Laser erfolgen.

Die Risse können grundsätzlich auf beliebige Art detektiert werden, beispielsweise akustisch oder durch Schwingungsanalysen. Risse im Inneren von Körpern können mit Ultraschall oder Röntgenstrahlung detektiert werden. Bei einer Prüfung einer Oberfläche eines Körpers ist es aber besonders bevorzugt und einfach, wenn die Risse optisch detektiert werden. Hierfür können die Risse mit einer Risseindringfarbe kenntlich gemacht werden. Dabei wird der zu prüfende Körper beispielsweise in eine gefärbte Flüssigkeit getaucht. Die gefärbte Flüssigkeit verbleibt nach Entnahme des Körpers aus der Flüssigkeit in den Rissen und bildet diese nach. Hierfür ist mit Vorteil vorgesehen, dass die Risse mit einer fluoreszierenden Risseindringfarbe versehen werden, worauf die Risse unter ultraviolettem Licht sichtbar gemacht werden. Der oder die zu prüfenden Körper brauchen dann bloß in eine Flüssigkeit getaucht werden und können anschließend sofort inspiziert werden. Körper mit zu großen Rissen werden dann ausgeschieden. Dieses Verfahren ist vor allem zur Prüfung von Rissen an der Oberfläche geeignet.

Das erfindungsgemäße Verfahren eignet sich insbesondere auch dafür, in einer laufenden Produktion eingesetzt zu werden. Hierfür kann vorgesehen sein, dass der Körper in einem Ofen im Durchlauf auf die erste Temperatur erwärmt wird. Dazu kann beispielsweise ein Fördermittel vorgesehen sein, mit dem der Körper durch den Ofen transportiert wird. Anschließend kann der Körper mit dem Fördermittel zu einem Abschreckmittel geführt werden. Als Abschreckmittel eignet sich insbesondere ein Fluid wie Wasser oder Öl. Möglich ist aber auch eine Abschreckung mit einem Gas. Bei einer Abschreckung mit Gas kann das Gas mit erhöhtem Druck auf den Körper geführt werden.

Wird der oder die Körper mit einem Fördermittel zu einem Fluid als Abschreckmittel geführt, kann der bzw. die Körper einfach in das Fluid fallen gelassen werden, um die Thermoschockbehandlung zu erreichen. Das Fluid weist dabei eine gegebene zweite Temperatur auf.

Möglich ist es im Rahmen der Erfindung auch, dass die Thermoschockbehandlung mit einer Erwärmung und einem anschließenden Quenchen in einer einzelnen Vorrichtung durchgeführt wird, insbesondere wenn mit einem Gas für eine Abschreckung von einer ersten Temperatur gearbeitet wird. Ein Erwärmen kann dabei unter beliebig einstellbarer Atmosphäre, aber auch unter Vakuum erfolgen. Bei einem Quenchen mittels eines Gases ist es bevorzugt, dass das Gas unter Hochdruck auf den Körper aufgebracht wird, z. B. mit einem Druck von 2 bar oder mehr.

In einer weiteren Verfahrensvariante wird bei der Thermoschockbehandlung ein inhomogenes Temperaturfeld am und/oder im Körper eingestellt, was sich durch ein rasches Erwärmen in Kombination mit einem raschen Abkühlen erreichen lässt.

Die Thermoschockbehandlung kann sich auf eine oberflächennahe Zone des Körpers oder den gesamten Körper beziehen. Möglich ist es dabei auch, dass die Thermoschockbehandlung in einem Bereich durchgeführt wird, in welchem eine maximale Belastung des Körpers im Einsatz gegeben ist. Insbesondere können auch Kanten eines Körpers der Thermoschockbehandlung unterworfen werden.

Weitere Merkmale, Vorteile und Wirkungen der Erfindung ergeben sich aus den nachfolgend dargestellten Ausführungsbeispielen. In den Zeichnungen, aufweiche dabei Bezug genommen wird, zeigen:

Fig. 1 eine Vorrichtung zur Thermoschockbehandlung;
Fig. 2 eine schematische Darstellung eines Temperaturverlaufs bei einer Thermoschockbehandlung mit einer Vorrichtung gemäß Fig. 1;
Fig. 3 eine schematische Darstellung der Inspektion von Körpern mit Risseindringfarbe.

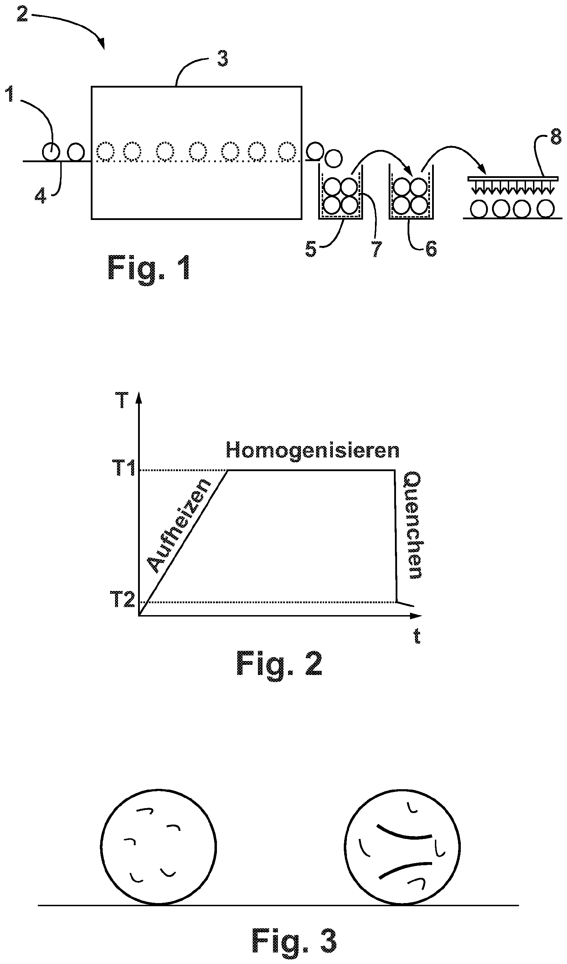

In Fig. 1 ist eine Vorrichtung 2 dargestellt, die insbesondere zur Durchführung eines erfindungsgemäßen Verfahrens im kontinuierlichen Durchlauf ausgelegt ist. Die Vorrichtung 2 umfasst einen Ofen 3, der als Durchlaufofen ausgebildet ist. Innerhalb des Ofens 3 ist ein Fördermittel 4, beispielsweise ein temperaturbeständiges Förderband, geführt. Auf dem Fördermittel 4 werden nacheinander mehrere keramische Körper 1 durch den Ofen 3 geführt. An den Ofen 3 schließt ein Behälter 5 an, in dem sich Wasser oder ein anderes Fluid als Abschreckmittel befindet. Die Körper 1 werden im Ofen 3 bei Durchlauf auf eine vorbestimmte erste Temperatur T1 gebracht, bis diese letztlich spätestens am Ende des Ofens 3 homogen eine erste Temperatur T1 aufweisen. Anschließend werden die Körper 1 in das Abschreckmittel fallen gelassen, das eine vorbestimmte zweite Temperatur T2 aufweist, die niedriger als die erste Temperatur T1 ist. Dadurch wird eine Thermoschockbehandlung herbeigeführt. Um die Körper 1 auf einfache Weise unter fortlaufenden Betrieb aus dem Behälter 5 entnehmen zu können, ist in diesem ein Sieb 7 oder ein anderes Hilfsmittel angeordnet. Ist das Sieb 7 mit Körpern 1 gefüllt, werden diese mit dem Sieb 7 entnommen und in einen weiteren Behälter 6 getaucht, der eine Lösung mit einer fluoreszierenden Risseindringfarbe aufweist. Nach kurzem Eintauchen werden die Körper 1 aus diesem weiteren Behälter 6 entnommen und anschließend unter einer UV-Lichtquelle 8 optisch auf überkritisch lange Risse untersucht. Körper 1, die eine zulässige maximale Rissgröße überschreiten, werden dabei ausgeschieden.

In Fig. 2 ist stark schematisiert ein Temperaturverlauf der Thermoschockbehandlung dargestellt. In einer ersten Phase wird der oder die Körper 1 auf eine vorbestimmte Temperatur T1 erwärmt. Nachdem der oder die Körper 1 homogen die erste Temperatur T1 aufweisen, können diese in einem oder mit einem Medium wie ein Fluid oder Gas auf eine zweite Temperatur T2 abgeschreckt werden. Eine Temperaturdifferenz zwischen erster Temperatur T1 und zweiter Temperatur T2 ist dabei so ausgelegt, dass diese mit einer vorgegebenen Toleranz nach oben einer mechanischen Belastung eines Körpers 1 im Einsatz entspricht.

Bei den Körpern 1 kann es sich um beliebige keramische Körper 1 handeln. Insbesondere bewährt sich das Verfahren jedoch bei keramischen Körpern 1, die für einen Einsatz besonders hohe Güte an der Oberfläche aufweisen sollen, weil ein Materialversagen zu Komplikationen führen kann, die nur aufwendig zu beheben sind. Hierbei kann es sich beispielsweise um keramische Implantate wie künstliche Gelenke, Zahnprothesen oder Zahnbrücken handeln, die bei Versagen eventuell eine zusätzliche Operation des Patienten erfordern. Andere Anwendungsbeispiele betreffen Wälzkörper für Wälzlager, die bei Materialversagen aufwendige Reparaturarbeiten bedingen. Darüber hinaus können auch andere Bauteile wie Ventile, Düsenkörper, Werkzeugteile oder keramische Leiterplatten, funktionelle Komponenten etc. geprüft werden. Manche der Komponenten können dabei auch metallische oder organische Materialien enthalten.

In Fig. 3 ist ebenfalls schematisiert dargestellt, wie sich die Risseindringfarbe nach der Thermoschockbehandlung auf Körpern 1 darstellt. Während ein linker Körper 1 nur kleine und damit akzeptable Risse aufweist, die während der Thermoschockbehandlung auch nicht merklich gewachsen sind, weist ein rechts dargestellter Körper 1 zwei größere Risse auf, die im Einsatz zu einem Materialversagen führen würden, weil die Risse nach der Thermoschockbehandlung bereits überkritisch lang sind. Daher wird der rechte Körper 1 ausgeschieden, wohingegen der linke Körper 1 einsatztauglich ist.

Mit einem Verfahren wie vorstehend dargestellt können beispielsweise keramische Körper 1 aus Siliciumnitrid geprüft werden, ehe diese in Kugellagern verbaut werden. Hierfür werden die Körper 1 wie beschrieben einer Prüfung mittels Thermoschock unterzogen und anschließend mit Risseindringfarbe beaufschlagt, um überkritisch lange Risse zu detektieren und damit schadhafte bzw. nicht einsatztaugliche Körper 1 ausscheiden zu können. Bei einer kugelförmigen Geometrie der Körper 1 und einem Durchmesser von ca. 12,7 mm kann für eine gewünschte bzw. geforderte Mindestfestigkeit von beispielsweise 700 MPa ein Toleranzbereich von 70 MPa vorgesehen werden, sodass die Belastung auf 770 MPa ausgelegt wird. Zur Berechnung der hierfür erforderlichen Temperaturdifferenz wird von den Materialeigenschaften wie Dichte und Wärmeleitfähigkeit ausgegangen und die Wärmeübertragungskoeffizienten berücksichtigt.

Erfindungsgemäß erfolgt für komplexere Geometrien eine nummerische Berechnung, wobei sich bei einem vorausgesetzten temperaturunabhängigen Wärmeübergangskoeffizienten in Abhängigkeit vom Material als Faustregel ergibt, dass bei größeren Körpern 1 eine Temperaturdifferenz von 1 °C etwa 2 MPa bis 3 MPa entspricht. Je kleiner die Körper 1 aus Siliciumnitrid sind und je geringer die Abschreckintensität ist, desto geringer wird auch die pro Grad Temperaturdifferenz im Körper 1 erzeugte Zugspannung im kälteren Probenteil. Im wärmeren Probenteil treten dagegen Druckspannungen in ähnlicher Höhe wie die Zugspannungen im kälteren Probenteil auf.

Es versteht sich, dass eine Prüfung auch auf andere Weise durchgeführt werden kann. Beispielsweise können Körper 1 bereichsweise oder vollständig mit einem Gasbrenner erwärmt und anschließend mit einem Luftjet abgekühlt werden. Auch kann sich eine Thermoschockbehandlung auf einzelne Bereiche eines Körpers 1 beschränken, der im Einsatz besonders hohen Belastungen unterliegt.

Neben der Prüfung mittels eines Durchlaufofens mit anschließendem Quenchen in einem externen Aggregat ist auch eine Prüfung in einer kombinierten Erwärmungs- und Abschreckanlage vorteilhaft. Ein Beispiel hierfür ist die Erwärmung in einem Vakuumhärteaggregat, wobei bei der Erwärmung mit oder ohne Vakuum gearbeitet werden kann, mit nachfolgender Hochdruckgasabschreckung.

## Patentansprüche

1. Verfahren zur Prüfung eines Körpers (1) mit sprödem Materialverhalten und mit komplexer Geometrie, insbesondere eines keramischen Körpers (1), auf Einsatztauglichkeit, wobei der Körper (1) zumindest bereichsweise einer Thermoschockbehandlung durch Abkühlung oder Erwärmung von einer ersten Temperatur (T1) auf eine zweite Temperatur (T2) unterworfen und vorhandene Risse detektiert werden, wonach auf Grundlage der detektierten Risse über die Einsatztauglichkeit des Körpers (1) entschieden wird, **dadurch gekennzeichnet, dass** auf Basis einer geforderten Bruchfestigkeit des Körpers (1) im Einsatz eine minimale Temperaturdifferenz zwischen der ersten Temperatur (T1) und der zweiten Temperatur (T2) für die Thermoschockbehandlung ermittelt und der Körper (1) anschließend der Thermoschockbehandlung mit zumindest der minimalen Temperaturdifferenz unterworfen wird, wobei die minimale Temperaturdifferenz nummerisch berechnet wird.

2. Verfahren nach Anspruch 1, **dadurch gekennzeichnet, dass** die Temperaturdifferenz so gewählt wird, dass die Bruchfestigkeit des Körpers (1) im Einsatz mit einer Toleranz überschritten wird, insbesondere einer Toleranz von maximal 20 %.

3. Verfahren nach Anspruch 1 oder 2, **dadurch gekennzeichnet, dass** der Körper (1) so lange auf die erste Temperatur (T1) erwärmt wird, bis dieser in erwärmten Bereichen homogen die erste Temperatur (T1) aufweist, und anschließend rasch abgekühlt wird.

4. Verfahren nach einem der Ansprüche 1 bis 3, **dadurch gekennzeichnet, dass** die Risse optisch detektiert werden.

**5.** Verfahren nach Anspruch 4, **dadurch gekennzeichnet, dass** die Risse mit einer Risseindringfarbe kenntlich gemacht werden.

**6.** Verfahren nach Anspruch 4 oder 5, **dadurch gekennzeichnet, dass** die Risse mit einer fluoreszierenden Risseindringfarbe versehen werden, worauf die Risse unter ultraviolettem Licht sichtbar gemacht werden.

**7.** Verfahren nach einem der Ansprüche 1 bis 6, **dadurch gekennzeichnet, dass** der Körper (1) in einem Ofen (3) im Durchlauf auf die erste Temperatur (T1) erwärmt wird.

**8.** Verfahren nach Anspruch 7, **dadurch gekennzeichnet, dass** der Körper (1) auf einem Fördermittel (4) durch den Ofen (3) transportiert wird.

**9.** Verfahren nach Anspruch 8, **dadurch gekennzeichnet, dass** der Körper (1) mit dem Fördermittel (4) zu einem Abschreckmittel geführt wird.

**10.** Verfahren nach Anspruch 9, **dadurch gekennzeichnet, dass** das Abschreckmittel ein Fluid ist.

**11.** Verfahren nach einem der Ansprüche 1 bis 6, **dadurch gekennzeichnet, dass** die Thermoschockbehandlung mit einer Erwärmung und einem anschließenden Quenchen in einer einzelnen Vorrichtung durchgeführt wird.

**12.** Verfahren nach Anspruch 11, **dadurch gekennzeichnet, dass** das Quenchen mittels eines Gases unter Hochdruck erfolgt.

**13.** Verfahren nach einem der Ansprüche 1 oder 2, **dadurch gekennzeichnet, dass** bei der Thermoschockbehandlung ein inhomogenes Temperaturfeld am und/oder im Körper (1) eingestellt wird.

**14.** Verfahren nach einem der Ansprüche 1 bis 13, **dadurch gekennzeichnet, dass** die Thermoschockbehandlung in einem Bereich durchgeführt wird, in welchem eine maximale Belastung des Körpers (1) im Einsatz gegeben ist.

**15.** Verfahren nach einem der Ansprüche 1 bis 14, **dadurch gekennzeichnet, dass** Kanten eines Körpers (1) der Thermoschockbehandlung unterworfen werden.

**Claims**

**1.** A method for testing a body (1) with brittle material behaviour and with complex geometry, particularly a ceramic body (1), for suitability for use, the body (1) being subjected at least in certain regions to a thermal shock treatment by cooling or heating from a first temperature (T1) to a second temperature (T2) and cracks that are present being detected, according to which, a decision is made about the suitability for use of the body (1) on the basis of the detected cracks, **characterized in that**, on the basis of a required breaking strength of the body (1) in use, a minimum temperature difference between the first temperature (T1) and the second temperature (T2) is determined and the body (1) is subsequently subjected to the thermal shock treatment using at least the minimum temperature difference, wherein the minimum temperature difference is calculated numerically.

**2.** The method according to claim 1, **characterized in that** the temperature difference is chosen in such a manner that the breaking strength of the body (1) in use is exceeded with a tolerance, particularly a tolerance of at most 20%.

**3.** The method according to claim 1 or 2, **characterized in that** the body (1) is heated to the first temperature (T1) until the body exhibits the first temperature (T1) homogeneously, and is subsequently cooled rapidly.

**4.** The method according to one of claims 1 to 3, **characterized in that** the cracks are detected optically.

**5.** The method according to claim 4, **characterized in that** the cracks are indicated using a crack penetration colour.

**6.** The method according to claim 4 or 5, **characterized in that** the cracks are provided with a fluorescent crack penetration colour, whereupon the cracks are made visible under ultraviolet light.

**7.** The method according to one of claims 1 to 6, **characterized in that** the body (1) is heated to the first temperature (T1) in an oven (3) in continuous operation.

8. The method according to claim 7, **characterized in that** the body (1) is transported through the oven (3) on a conveyor (4).

9. The method according to claim 8, **characterized in that** the body (1) is conveyed to a quenching medium using the conveyor (4).

10. The method according to claim 9, **characterized in that** the quenching medium is a fluid.

11. The method according to any one of claims 1 to 6, **characterized in that** the thermal shock treatment is carried out using heating and subsequent quenching in a separate device.

12. The method according to claim 11, **characterized in that** the quenching takes place by means of a gas under high pressure.

13. The method according to one of claims 1 or 2, **characterized in that** an inhomogeneous temperature field is set up on and/or in the body (1) during the thermal shock treatment.

14. The method according to one of claims 1 to 13, **characterized in that** the thermal shock treatment is carried out in a region in which there is a maximum load of the body (1) in use.

15. The method according to one of claims 1 to 14, **characterized in that** edges of a body (1) are subjected to the thermal shock treatment.


**Revendications**

1. Procédé de contrôle d'un corps (1) avec comportement cassant de matériau et avec géométrie complexe, en particulier d'un corps céramique (1) en matière d'aptitude à l'utilisation, sachant que le corps (1) est soumis au moins par zones à un traitement par choc thermique par refroidissement ou réchauffement d'une première température (T1) à une deuxième température (T2) et des fissures présentes sont détectées, après quoi l'aptitude à l'utilisation du corps (1) est décidée sur la base des fissures détectées, **caractérisé en ce que** sur la base d'une résistance à la rupture exigée du corps (1) en utilisation, une différence de température minimale est déterminée entre la première température (T1) et la deuxième température (T2) pour le traitement par choc thermique et le corps (1) est ensuite soumis au traitement par choc thermique avec au moins la différence de température minimale, sachant que la différence de température minimale est calculée de façon numérique.

2. Procédé selon la revendication 1, **caractérisé en ce que** la différence de température est choisie de telle sorte que la résistance à la rupture du corps (1) est dépassée en utilisation avec une tolérance, en particulier avec une tolérance maximale de 20 %.

3. Procédé selon la revendication 1 ou 2, **caractérisé en ce que** la corps (1) est chauffé à la première température (T1) aussi longtemps que celui-ci comporte la première température (T1) de façon homogène dans les zones chauffées et est ensuite rapidement refroidi.

4. Procédé selon l'une quelconque des revendications 1 à 3, **caractérisé en ce que** les fissures sont détectées de manière optique.

5. Procédé selon la revendication 4, **caractérisé en ce que** les fissures peuvent être identifiées avec un colorant de pénétration des fissures.

6. Procédé selon la revendication 4 ou 5, **caractérisé en ce que** les fissures sont dotées d'un colorant de pénétration des fissures fluorescent, avec lequel les fissures peuvent être visualisées sous une lumière ultraviolette.

7. Procédé selon l'une quelconque des revendications 1 à 6, **caractérisé en ce que** le corps (1) est chauffé dans un four (3) au cours d'un passage à la première température (T1).

**8.** Procédé selon la revendication 7, **caractérisé en ce que** le corps (1) est transporté à travers le four (3) sur un moyen de transport (4).

**9.** Procédé selon la revendication 8, **caractérisé en ce que** le corps (1) est guidé avec le moyen de transport (4) vers un milieu de refroidissement.

**10.** Procédé selon la revendication 9, **caractérisé en ce que** le milieu de refroidissement est un fluide.

**11.** Procédé selon l'une quelconque des revendications 1 à 6, **caractérisé en ce que** le traitement par choc thermique est exécuté avec un réchauffement et un refroidissement rapide consécutif dans un dispositif individuel.

**12.** Procédé selon la revendication 11, **caractérisé en ce que** le refroidissement rapide a lieu au moyen d'un gaz sous haute pression.

**13.** Procédé selon l'une quelconque des revendications 1 ou 2, **caractérisé en ce que** lors du traitement par choc thermique, un champ de températures inhomogène est instauré sur et/ou dans le corps (1).

**14.** Procédé selon l'une quelconque des revendications 1 à 13, **caractérisé en ce que** le traitement par choc thermique est exécuté dans une zone dans laquelle une sollicitation maximale du corps (1) est mise en œuvre.

**15.** Procédé selon l'une quelconque des revendications 1 à 14, **caractérisé en ce que** les bords d'un corps (1) sont soumis au traitement par choc thermique.

**Fig. 1**

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP H0886731 A
- DE 102010017351 A1